# EUROPÄISCHE PATENTSCHRIFT

(11) **EP 1 838 301 B1**
(45) Veröffentlichungstag und Bekanntmachung des Hinweises auf die Patenterteilung: **28.01.2015**
(21) Anmeldenummer: 04802396.4
(22) Anmeldetag: 28.12.2004
(51) Int. Cl.: A61K 31/357, A61P 23/00

(54) **VERWENDUNG VON RESINIFERATOXIN (RTX) FÜR DIE HERSTELLUNG EINES MITTELS ZUR BEHANDLUNG VON SCHMERZEN**
USE OF RESINIFERATOXIN (RTX) FOR THE PREPARATION OF AN AGENT FOR THE TREATMENT OF PAIN
UTILISATION DE LA RESINIFERATOXINE (RTX) POUR LA PRODUCTION D'UN AGENT DESTINÉ AU TRAITEMENT DES DOULEURS

(43) Veröffentlichungstag der Anmeldung: 03.10.2007
(73) Patentinhaber: Mestex AG, 8008 Zürich (CH)
(72) Erfinder: MEYER, Dominik, CH-8008 Zürich (CH)
(74) Vertreter: Lusuardi, Werther
(86) Internationale Anmeldenummer: PCT/CH2004/000756
(87) Internationale Veröffentlichungsnummer: WO 2006/069451

(56) Entgegenhaltungen:
- WO-A-2004/058286
- WO-A1-2006/066419
- US-A1- 2003 104 085
- KARAI L ET AL: "Deletion of vanilloid receptor 1-expressing primary afferent neurons for pain control" JOURNAL OF CLINICAL INVESTIGATION, NEW YORK, NY, US, Bd. 113, Nr. 9, 2004, Seiten 1344-1352, XP002333034 ISSN: 0021-9738

## Beschreibung

Die Erfindung bezieht sich auf die Verwendung von Resiniferatoxin (RTX) für die Herstellung eines Mittels zur Behandlung von Schmerzen gemäss dem Oberbegriff des Patentanspruchs 1 und ein Mittel zur Verwendung bei der Behandlung von Schmerzen gemäss dem Oberbegriff des Patentanspruchs 21.

Von Gelenken ausgehende Schmerzen haben ihren Ursprung häufig im Bereich der Gelenkkapsel oder im gelenknahen Bereich des Knochens. Dabei können viele Ätiologien in Frage kommen, z.B. arthrotische oder arthritische Krankheitsformen, mechanische oder andere Reizung der gelenknahen Knochenoberfläche, Reizung oder Verletzung der Gelenksbandstrukturen, Infekte, autoimmune Prozesse, u.s.w.. In allen Fällen, welche im Rahmen dieser Erfindung von Interesse sind, gehen die entstehenden Schmerzen von nociceptiven Nervenfasern im gelenknahen Bereich aus. Nociceptive Nervenfasern werden auch als C-Fasern und A-delta Fasern bezeichnet. Wird in ein so erkranktes Gelenk eine analgetische Substanz (z.B. Lokalanästhetika oder Morphine) injiziert, so werden die Beschwerden des Patienten gelindert. Allerdings haben die heute gebräuchlichen Substanzen eine nur beschränkte Wirkdauer, weshalb die Beschwerden meistens zurückkehren.

Zur Therapie schmerzhaft erkrankter Gelenke werden heute generell folgende Verfahren angewendet:
- Physiotherapie/Bewegungstherapie
- Systemische analgetische/antiphlogistische Therapie (etc.)
- Lokale analgetische/antiphlogistische Verfahren (etc.)
- Operative Verfahren:
- Arthroskopisch: Debridement, Gelenkstoilette, etc.
- Offen/Mini-offen: Gelenksersatz, Gelenkversteifung, etc.

In der Literatur wurden auch schon eine Reihe von bekannten Substanzen zur Therapie schmerzhafter, entzündlicher Gelenke vorgeschlagen, insbesondere:
- Osmiumsäure oder radioaktive Substanzen wie Technetium 99, welche zu einer Synoviorthese führen;
- Injektion von Lokalanästhetika, Hyaluronsäurepräparaten (etc.)
- Injektion von Antiphlogistika
- Injektion von Kontrastmitteln zur Gelenksdiagnostik
- Gelenkspülung zur Gelenkstoilette
- Chemische, thermische, elektrische oder chirurgische Ablation der gelenksversorgenden Nerven.

Alle bisher verwendeten Substanzen und Verfahren führen nur zu einer relativ kurzfristigen oder unvollständigen Schmerzfreiheit oder verursachen bleibende Schädigungen am Gelenk.

So besteht beispielsweise beim bekannten Verfahren der Synoviorthese der Nachteil der Zerstörung der molekularen Strukturen, insbesondere Denaturierung der Proteine, welche im Prozess der Arthritis und z.T. auch Arthroseentwicklung als Entzündungsauslöser wirken. Dabei entsteht eine Fibrose der Gelenkkapsel welche weniger entzündlich und somit auch weniger schmerzhaft ist. Gleichzeitig wird durch die bei der Synoviorthese auftretende Fibrose des Gelenkes die zumeist vorhandene und dabei ebenfalls zu behandelnde Hyperämie vermindert, woraus sich ebenfalls therapeutischer Nutzen ergibt. Die fibrotische Vernarbung nach Synoviorthese kann aber zu einer verminderten Beweglichkeit des Gelenkes führen, sowie zu einer verminderten Produktion von Synovialflüssigkeit und zur Zerstörung des Gelenkknorpels. Diese unerwünschte Fibrose der Gelenkskapsel sollte vermieden und nur die sensible Innervation des Gelenkes ausgeschaltet werden.

Aus der EP-B 0 998 288 CAMPBELL ist die Verwendung von Capsaicin und Analogen davon zusammen (simultan oder sequentiell) mit einem Lokalanästhetikum bekannt.
Lokalanästhetika sollen dabei brennende Schmerzen bei der Injektion von RTX verhindern. Weil die Lokalanästhetika aber eine antagonistische Wirkung gegenüber Capsaicinen haben, muss in Kombination von Lokalanästhetika mit Capsaicinen eine höhere Konzentration an Capsaicinen gewählt werden als dies mit Capsaicinen allein nötig wäre, um die gewünschte Schmerztherapie zu erzielen. Capsaicine bewirken als Nebenwirkung eine Hyperämie und Entzündungsreaktion des Gewebes.
Auch aus der US 4,997,853 BERNSTEIN ist die Verwendung von Capsaicin zusammen mit einem oberflächlich wirkenden Lokalanästhetikum zur Behandlung von oberflächlichen Schmerzsyndromen bekannt.
Die Verwendung von Capsaicinen ohne Lokalanästhetika ist zwar bekannt bei systemischer Anwendung (Applikation intraperitoneal, subcutan, intravenös usw.) oder bei regionaler Anwendung (Applikation epidural, intrathekal, transcutan, oder als regionaler selektiver Nervenblock), jedoch immer in Kombination mit einer AllgemeinAnästhesie der Versuchstiere. Der entscheidende Nachteil einer regionalen oder systemischen Anwendung ist aber, dass nicht nur das betroffene Gebiet behandelt wird, sondern auch asymtomatische, danebenliegende Gebiete.
Die Anwendung von Capsaicinen in der Blase (intravesikal) ist zwar ebenfalls ohne Lokalanästhesie bekannt, hier wird das Mittel aber nur oberflächlich angewandt und nicht durch eine Hautbarriere injiziert.
Hier will die Erfindung Abhilfe schaffen. Der Erfindung liegt die Aufgabe zugrunde eine Verwendung von Resiniferatoxin (RTX) für die Herstellung eines Mittels zur Behandlung von Gelenkschmerzen gemäss dem Oberbegriff des Anspruchs 1 und ein Mittel zur Verwendung bei der Behandlung von Schmerzen gemäss dem Oberbegriff des Anspruchs 21 bereitzustellen, welche die für die Nociception verantwortlichen Nervenendigungen zur längerdauernden Analgesie dauerhaft schädigen, ohne die gelenkfernen Strukturen zu gefährden.
Durch alleinige Verwendung von RTX (vorzugsweise mit einer speziellen Konzentration), d.h. ohne Allgemeinanästhesie des Patienten wurde überraschenderweise die gleiche oder bessere Wirkung erzielt als bei Anwendung einer Allgemeinanästhesie.

Die Erfindung löst die gestellte Aufgabe mit der Verwendung von Resiniferatoxin (RTX) gemäss den Merkmalen des Anspruchs 1 und einem Mittel gemäss den Merkmalen des Anspruchs 21

Das erfindungsgemässe Mittel zur Verwendung bei der Behandlung von Schmerzen ist dadurch gekennzeichnet, dass Resiniferatoxin (RTX) in ein schmerzhaftes oder erkranktes Gelenk des Körpers bei Mensch oder Tier lokal injiziert wird. Das RTX kann entweder dort belassen werden oder nach einer gewissen Einwirkzeit wieder vollständig oder teilweise abgesogen werden. Das RTX diffundiert nun zu den sensiblen Nervenendigungen, welche direkt oder indirekt den Bereich des Gelenkes innervieren, hemmt oder schädigt diese prädominant und führt damit zu einer verminderten Wahrnehmung der Gelenkschmerzen. Neu dabei ist ferner, dass die Gelenkskapsel oder der Gelenkschmierbeutel dazu verwendet wird die Wirkung des RTX auf den Ort der Schmerzentstehung zu konzentrieren und dadurch lokal eine höhere Konzentration von RTX zu erlauben, als es ohne die schützende Gelenkskapsel oder den Gelenkschmierbeutel in der gleichen Konzentration und Verträglichkeit möglich wäre und gleichzeitig die Gefäss-/Nervenstrukturen und andere Strukturen in der Nähe des Gelenkes verhältnismässig zu schonen. Somit wird eine langfristige Linderung der vom dem erkrankten Band-Kapsel-Gelenk-Komplex ausgehenden Schmerzempfindung durch Hemmung oder Ausschaltung der Reizleitung erlangt. Dieses Verfahren kann sowohl präventiv oder therapeutisch angewandt werden.

Die Vorteile der erfindungsgemässen Verwendung von RTX und des dabei anwendbaren Verfahrens sind die folgenden:
- Das angenehme warme Gefühl bei Injektion ohne Narkose hat einen entscheidenden unterstützenden positiven Effekt für die gewünschte Wirkung.
- Die intraartikuläre Injektion von RTX zur analgetischen Therapie von Gelenken führt zu einer weitgehenden Schonung der Kapsel-Bandstrukturen, der Synovia und der Knorpel-Knochenstrukturen und somit zur Erhaltung der physiologischen Verhältnisse.
- Die Nutzung der Gelenkskapsel als natürliche Grenze der Verteilung des RTX.
- Die Wirkungsentfaltung des RTX ist nicht von spezifischen neuronalen Epitopen ausser dem TRPV1 Rezeptor abhängig.
- Das Verfahren ist durch Nicht-Spezialisten durchführbar.
- Das Verfahren ist mit einer dünnen, auch nicht-arthroskopischen Nadel durchführbar.
- Das Verfahren ist nicht infektionsgefährdend, im Gegensatz zum beliebten Verfahren der Cortisoninjektion, welches stark lokal infektionsfördernd ist, da Cortison lokal das Immunsystem hemmt.
- Das Verfahren führt zu einer lokalen sensiblen Denervation, d.h. einer Ausschaltung von schmerzleitenden Nerven.
- Erweiterung der Gelenksbeweglichkeit durch Aufhebung der schmerzhaften Bewegungseinschränkung im Gegensatz zur Synoviorthose, bei welcher durch die entstehende Kapselfibrose eine Bewegungseinschränkung erfolgt.
- Positive Vorbereitung für eine spätere Arthroplastik. Durch die sklerotisierende Wirkung von RTX (einerseits als Folge einer chemisch-biologischen Reaktion anderseits durch die mechanische Belastung bei der schmerzfreien Gelenksbenutzung) erhält der gelenknahe Knochen eine für den späteren Halt einer Prothese vorteilhaftere Struktur.
- Keine lokale Fettgeweberesorption (Lipolyse)
- Keine Schwächung von kollagenen Sehnen/Band/Kapsel-Strukturen.

Die Erfindung wird im Folgenden für die Anwendung beim Menschen beschrieben, insbesondere beziehen sich die angegebenen Dosierungen auf die Humanapplikation. Die Erfindung eignet sich aber auch für den Veterinärbereich, wobei dort Anpassungen in der Dosierung vorgenommen werden müssen in Abhängigkeit vom Körpergewicht des jeweiligen Tieres.

Bei einer besonderen Ausführungsform bezieht sich die Verwendung von Resiniferatoxin (RTX) - ohne Beigabe von weiteren pharmakologisch wirksamen Substanzen - auf die Herstellung eines Mittels zur Behandlung von lokalen Schmerzen, insbesondere von
a) lokalen Wundschmerzen nach OP in Form einer Spüllösung zur intraoperativen Anwendung bei einer offenen oder arthroskopischen oder endoskopischen Operation oder Liposuction;
b) lokale Behandlung von Gelenkschmerzen durch intraartikuläre Injektion bei
   Chondrocalzinose
   Ligamentären Schäden
   Meniskusläsion
   Knorpelschaden
   Synovitis
   Arthrofibrose
   Morbus Sudeck
   Nekrose der Gelenkanteile
   Neuropathischen Gelenkschmerzen
c) lokale Behandlung von Knochenschmerzen nach Knochen-Operation durch Auftragung auf den Knochen, z.B. nach
   Beckenkammosteotomie
   Hallux-Valgus Korrektur
d) Behandlung von Knochenschmerzen durch Injektion in den Knochen, insbesondere bei Femurkopfnekrose in den Femurkopf, in den Wirbelkörper bei Osteochondrose
e) Lokale Behandlung von Schmerzen bei Gelenksteife, insbesondere bei Arthrofibrose oder Frozen shoulder;
f) Lokale Behandlung von Muskelschmerzen, durch intramuskuläre Injektion, insbesondere bei Muskelfaserriss, Muskelkater, oder spastischen Erkrankungen;
g) Lokale Injektion in den schmerzhaften Meniskus bei Meniskusdegeneration oder Meniskusriss;
h) Behandlung von Rückenschmerzen durch Injektion in die Bandscheibe bei Bandscheibendegeneration oder Bandscheibenriss;
i) Injektion um einen schmerzhaften Nerven, insbesondere bei Trigeminusneuralgie, Neurinom, Morton Neurom, Phantomschmerz, Narbenneurom;
k) Behandlung von Zahnschmerzen durch lokale Anwendung *intra*/*peridental,* insbesondere bei Karies, allen Formen von Zahnschmerz, Vor/bei/nach Zahnextraktion, Vor/bei/nach Zahnimplantat, Lokale Anwendung bei Parodontitis, Lokale Anwendung bei freiliegenden Zahnhälsen;
l) Injektion in die Pleurahöhle bei pleuritischen Schmerzen;
m) Instillation in den Darm bei Darmschmerzen, insbesondere bei Colitis Ulcerosa, M.Crohn, Analfissur,

Im speziellen wird die Verwendung von Resiniferatoxin (RTX) für die Herstellung eines Mittels zur lokalen Behandlung von Schmerzzuständen vorgeschlagen, nämlich von:
- postoperativen Schmerzzustände
- Arthritis
- für lokale Wundschmerzen nach OP in Form einer Spüllösung zur intraoperativen Anwendung bei einer offenen oder arthroskopischen oder endoskopischen Operation, inklusive Liposuction;
   Gelenkschmerzen;
   Knochenschmerzen nach Osteotomie;
   frozen shoulder
- für lokale Wundschmerzen nach OP in Form einer Spüllösung zur intraoperativen Anwendung bei einer offenen oder arthroskopischen oder endoskopischen Operation, inklusive Liposuction
- Lokale Behandlung von Gelenkschmerzen durch intraarticuläre Injektion bei
   Arthrose
   Rheumatoider Arthritis
   Infektiöser Arthritis
   Chondrocalzinose
   Ligamentären Schäden
   Meniskusläsion
   Knorpelschaden
   Synovitis
   Arthrofibrose
   Morbus Sudeck
   Nekrose der Gelenkanteile
   Neuropathischen Gelenkschmerzen
Lokale Behandlung von Knochenschmerzen nach Knochen-Operation durch Auftragung
auf den Knochen, z.B. nach
Beckenkammosteotomie
Hallux-Valgus Korrektur
Behandlung von Knochenschmerzen durch Injektion in den Knochen
z.B. bei Femurkopfnekrose in den Femurkopf
in den Wirbelkörper bei Osteochondrose
Lokale Behandlung von Schmerzen bei Gelenksteife, vorzugsweise Arthrofibrose oder Frozen shoulder;
Lokale Behandlung von Muskelschmerzen, durch intramuskuläre Injektion, vorzugsweise bei Muskelfaserriss, Muskelkater oder spastischen Erkrankungen:
Lokale Injektion in den schmerzhaften Meniskus bei Meniskusdegeneration oder Meniskusriss Behandlung von Rückenschmerzen durch Injektion in die Bandscheibe bei Bandscheibendegeneration oder Bandscheibenriss Injektion um einen schmerzhaften Nerven, insbesondere bei Trigeminusneuralgie, Neurinom, Morton Neurom, Phantomschmerz und Narbenneurom;
Behandlung von Zahnschmerzen durch lokale Anwendung intra/peridental bei:
Karies
Allen Formen von Zahnschmerz
Vor/bei/nach Zahnextraktion
Vor/bei/nach Zahnimplantat
Lokale Anwendung bei Parodontitis
Lokale Anwendung bei freiliegenden Zahnhälsen.
Injektion in die Pleurahöhle bei pleuritischen Schmerzen

Die Konzentration an RTX beträgt zweckmässigerweise zwischen 100 nM bis 10µM, vorzugsweise zwischen 500nM bis 1µM.
Das Mittel enthält vorzugsweise keinen Alkohol, insbesondere kein Ethanol. Ethanol hat den Nachteil dass es eine lokale Entzündung bewirkt und zu einer schmerzhaften Neuritis führen kann.

Überraschenderweise wurde gefunden, dass eines der Capsaicin-Analogen, nämlich das Resiniferatoxin (RTX) bei lokaler Applikation in einer weit grösseren Verdünnung (in der Grössenordnung von 1:1000) als Capsaicin wirksam ist, wenn es ohne Lokalanästhetikum verwendet wird. Es tritt kein Brennen auf und auch keine Entzündung des Gewebes. Insbesondere die lokale intraartikuläre Injektion von RTX allein (in sehr tiefer Konzentration) hat sich als wirksamer erwiesen als zusammen mit einem Lokalanästhetikum. Sie ist zudem entzündungs- und schmerzfrei. Insbesondere kommt diese Zubereitung ohne die Verwendung von Ethanol aus, was bei der bekannten Anwendung intravesikal sonst immer notwendig ist. Ein weiterer überraschender Vorteil bei der Anwendung ohne Lokal- oder Allgemeinanästhesie ist, dass durch die Injektion ein angenehmes warmes Gefühl eintritt, welches die Schmerzbekämpfung entscheidend unterstützt. Auf diese Weise wird eine Schmerzlinderung erzielt, welche diejenige bei einer Kombination mit Lokalanästhetika weit übertrifft. Man kann dies auch durch den antagonistischen Effekt erklären, welche die Lokalanästhetika für die Vanilloidrezeptoragonisten haben, da sie den gewünschten neuro-toxischen Effekt von Vanilloiden teilweise blockieren.

Bei einer bevorzugten Ausführungsform der Erfindung wird zusätzlich zum RTX ein Röntgenkontrastmittel, vorzugsweise gadoliniumhaltige, jodhaltige oder bariumhaltige Substanzen, z.B. ein Bariumzusatz oder ein MRI-Kontrastmittel verwendet, so dass eine bildgebende Kontrolle der Verteilung des RTX im intrakapsulären Raum möglich ist. Als Kontrastmittel können je nach Verfahren folgende Substanzen verwendet werden:

| | |
|---|---|
| Röntgen, CT: | Jodhaltige Substanzen, z.B. trijodierte Benzoate oder lopamidol, idealerweise 30 - 80g/100ml oder |
| | z. B. 5 - 10% eines anderen Kontrastmittels, z.B. Barium. |
| MRI: | z.B. Gadolinium, z.B. pro 1ml: 469,01mg Gadopentat Dimeglumid, 0,99mg Meglumin, 0,4mg Diethylentriamin-pentaacetat. |

Bei einer weiteren Ausführungsform wird zusätzlich zum RTX eine antibiotische, desinfizierende und/oder sterilisierende Substanz beigefügt.

Bei einer weiteren Ausführungsform wird zusätzlich zum RTX ein visköser Zusatzstoff, z.B. Hyaluronsäure, vorzugsweise mit einer Konzentration von 0,1-10,0 mg/ml Injektionslösung verwendet, was zu einer mechanischen Gleitverbesserung des Gelenkes führt.

Bei einer weiteren Ausführungsform wird zusätzlich zum RTX ein Vasokonstriktor verwendet, vorzugsweise Adrenalin, Noradrenalin, Phenylephrin oder Ornipressin oder andere, ähnliche, vorzugsweise alpha-adrenerge Vasokonstriktoren. Mit Adrenalin kann die Gesamtdosis des Neurotoxins (d.h. für das periphere Nervensystem toxische Substanz) um zirka den Faktor 2 gesteigert werden, da so die systemische Wirkung durch die verminderte Resorption reduziert wird. Die Adrenalinkonzentration kann 1:10'000 bis 1:80'000 bis 1:200'000 betragen. Die Gesamtdosis an Adrenalin liegt bei < 0,25 mg. Eine 50 ml Lösung von 1:200'000 Adrenalin enthält 0,25 mg Adrenalin.

Bei einer weiteren Ausführungsform wird zusätzlich zum RTX Glyzerin als Lösungsmittel verwendet. Glyzerin besitzt ebenfalls neurotoxische Eigenschaften (insbesondere aber wenn es intraneural gespritzt wird). Im weiteren besitzt Glyzerin eine Schmierfähigkeit für das Gelenkes, so dass auch eine physikalische Wirkung auftritt. Die Konzentration an Glyzerin beträgt vorzugsweise zwischen 10 und 95 %. Als Lösungsmedium kann anstelle von Glyzerin auch Wasser, Kochsalzlösung, Sodiumiothalamate, lophendylat, Ricin, Poly-Ethlenglycol oder Propylenglycol verwendet werden. Der Vorteil von Glyzerin als Lösungsmittel ist, das dieses hyperbar und an sich auch schon etwas neurotoxisch ist.

Bei einer weiteren bevorzugten Ausführungsform wird zusätzlich zum RTX ein Steroid verwendet, um beim allfälligen Auftreten einer entzündlichen Reaktion diese zu kontrollieren. Ausserdem kann man damit eine eher kausale Behandlung von schmerzhaften, entzündlichen Gelenkserkrankungen hinzufügen, welche die symptomatische, neurolytische Therapie unterstützt. Als besonders geeignet hat sich Betamethason erwiesen; z.B. 5 mg Betamethason als Diproprionat (kristalline Suspension) und 2 mg Betamethason als Dinatriumphosphat (Lösung in 1 ml, kann der total zu injizierenden Menge beigefügt werden). Diese Lösung ist äquivalent zu 45/23 mg Prednison/Prednisolon.

Das Mittel wir vorzugsweise zur Denervation oder Neurolyse in degenerativ erkrankten Gelenken verwendet.

Das Mittel kann in einer Trägerflüssigkeit (Carrier), einem pharmakologisch akzeptablem Vehikel, insbesondere aus der Gruppe Natriumchlorid-Injektionslösung, Ringiers Injektionslösung, isotonische Dextrose, steriles Wasser Dextroselösung, Laktierte Ringers Injektionslösung, destilliertes Wasser oder Mischungen davon. zur lokalen Injektion aufgelöst sein.

Das Mittel kann zusätzlich einen Permeationsförderer, vorzugsweise Dimethylsulfoxid, Ethoxyethylendiglycol, Ethanol, Phosphatidylcholine, Propylenglykol dipelargonate (DPPG), oder glycolysierte ethoxylierte Glyceride enthalten.

Das Mittel kann zusätzlich auch eine Substanz enthalten, welche eine verzögerte und prolongierte Freisetzung des RTX ermöglicht, vorzugsweise Glucosaminoglycane oder Hyaluronsäure.
Das Mittel ist erfindungsgemäss in einer Pufferlösung mit einem pH-Wert höher als 7,6 vorzugsweise höher als 8,5 aufgelöst.
Als Lösungsmedium kann anstelle von Glyzerin auch Wasser, Kochsalzlösung, Sodiumiothalamate, lophendylat, Ricin, Poly-Ethlenglycol oder Propylenglykol verwendet werden. Der Vorteil von Glyzerin als Lösungsmittel ist, das dieses hyperbar und an sich auch schon etwas neurotoxisch ist.

Erfindungsgemäss wird zusätzlich zu RTX Calcium Ca²⁺ im Lösungsmittel verwendet. Calcium ist für die Wirkung von RTX notwendig und verbessert dessen Wirkung wenn in überphysiologischer Konzentration vorhanden. Die Konzentration an Calcium beträgt > 2 mMol, vorzugsweise > 4 mMol. Einige Stoffe haben sich ebenfalls als wirkungsverstärkend für RTX erwiesen, so z.B. Magnesium, Antioxidantien, Preservative und Excipienten, insbesondere Natriumbisulfit (> 0,2 %), NaHSO₃, Ammonium-Verbindungen, wie Ammoniumsulfat (NH₄)₂SO₄, 2 - 10 (-30%), Polysorbat 80 (PS80) 0,025 mg/ml.
Die im Lösungsmedium gelösten Salze und Ionen sind vorzugsweise höher als physiologisch normal (z.B in Ringer Lactat Lösung) konzentriert.

RTX ist in einem körperverträglichen Lösungsmittel gelöst und wird zweckmässigerweise in einer Volumenmenge injiziert, welche dem verfügbaren Platz im zu behandelnden Gelenk entspricht, so dass dieses sich leicht bis prall füllt. Damit wird der Vorteil einer optimalen lokalen Verteilung von RTX erreicht. Es ist aber auch möglich weniger Flüssigkeit zu injizieren, dann muss aber das Gelenk gut bewegt werden zur besseren Verteilung der Substanzkombination.
Das in den intrakapsulären Bereich zu injizierende Flüssigkeitsvolumen beträgt 0,1 bis 150 ml. Für ein Fingergelenk genügen etwa max. 1ml, für das Schultergelenk max. 10 ml, für das Kniegelenk max. etwa 30 - 50 ml, bevorzugt aber nicht über 2ml.

Die Dosierung der Substanzkombination hängt von der Lokalisation und Indikation ab. Die Dosierung des RTX hängt von der dessen absoluten Löslichkeit im gewählten Lösungsmedium ab. Einen wesentlichen Einfluss auf die Dosierung hat die Kapseldicke des betroffenen Gelenkes. Je dicker die Kapsel, desto höhere Konzentration oder Menge an RTX ist notwendig.

Ein geeignetes Verfahren zur Behandlung von Schmerzen mit dem erfindungsgemäss verwendeten Mittel bei der Behandlung von Schmerzen besteht darin, dass Resiniferatoxin (RTX) in einem geeigneten körperverträglichen Lösungsmittel gelöst wird und vorzugsweise ein Flüssigkeitsvolumen von 0,1 bis 150 ml davon
a) lokal in die schmerzbefallene Gewebestruktur des Patienten injiziert wird oder
b) b) lokal auf die Operationswunde geträufelt wird; oder
c) in den intrakapsulären Bereich lokal injiziert; oder
in den Gelenkschmierbeutel eines von Schmerzen betroffenen Gelenkes lokal injiziert wird.

Die nociceptiven Nervenfasern werden durch das Resiniferatoxin (RTX) für mindestens 14 Tage, vorzugsweise mindestens 8 Wochen schmerzunempfindlich gemacht. Das Resiniferatoxin (RTX) wird vorteilhafterweise in einer solchen Konzentration verwendet, dass eine Neurolyse auftritt. Die Konzentration des RTX liegt zweckmässigerweise zwischen ungefähr 10nanoMolar (nM) bis 100 MikroMolar (µM).

Das oben beschriebene Verfahren eignet sich insbesondere für folgende Indikationen:
- für lokale Wundschmerzen nach OP in Form einer Spüllösung zur intraoperativen Anwendung bei einer offenen oder arthroskopischen oder endoskopischen Operation sowie Liposuction;
- Lokale Behandlung von Gelenkschmerzen durch intraarticuläre Injektion bei
   Arthrose
   Rheumatoider Arthritis
   Infektiöser Arthritis
   Chondrocalzinose
   Ligamentären Schäden
   Meniskusläsion
   Knorpelschaden
   Synovitis
   Arthrofibrose
   Morbus Sudeck
   Nekrose der Gelenkanteile
   Neuropathischen Gelenkschmerzen
Lokale Behandlung von Knochenschmerzen nach Knochen-Operation durch Auftragung auf den Knochen, z.B. nach Beckenkammosteotomie oder Hallux-Valgus Korrektur;
Behandlung von Knochenschmerzen durch Injektion in den Knochen
z.B. bei Femurkopfnekrose in den Femurkopf
in den Wirbelkörper bei Osteochondrose
Lokale Behandlung von Schmerzen bei Gelenksteife, vorzugsweise bei Arthrofibrose oder Frozen shoulder;
Lokale Behandlung von Muskelschmerzen, durch intramuskuläre Injektion, vorzugsweise bei Muskelfaserriss, Muskelkater oder spastischen Erkrankungen; Lokale Injektion in den schmerzhaften Meniskus bei Meniskusdegeneration oder Meniskusriss Behandlung von Rückenschmerzen durch Injektion in die Bandscheibe bei Bandscheibendegeneration oder Bandscheibenriss Injektion um einen schmerzhaften Nerven, vorzugsweise bei Trigeminusneuralgie, Neurinom, Morton Neurom, Phantomschmerz oder Narbenneurom;
Behandlung von Zahnschmerzen durch lokale Anwendung intra/peridental bei:
Karies
Allen Formen von Zahnschmerz
Vor/bei/nach Zahnextraktion
Vor/bei/nach Zahnimplantat
Lokale Anwendung bei Parodontitis
Lokale Anwendung bei freiliegenden Zahnhälsen.
Injektion in die Pleurahöhle bei pleuritischen Schmerzen
Instillation in den Darm bei Darmschmerzen, insbesondere bei Colitis Ulcerosa, M.Crohn oder Analfissur.

Der Vorteil dieses Verfahren besteht darin, dass es eine lokale Anwendung ohne Lokalanästhetika erlaubt, wodurch eine geringere RTX-Konzentration möglich ist und dadurch weniger lokale Nebenwirkungen von RTX wie Schwellung oder Entzündung auftreten.

Bei einer besonderen Ausführungsform wird das Mittel in eine synoviale Höhle eingespritzt, welche nicht mit Urothel ausgekleidet ist. Die Injektion in eine synoviale Höhle hat sich als besonders vorteilhaft erwiesen, weil hier eine optimale Verweildauer zu Wirkungsentfaltung kombiniert mit minimalen Nebenwirkungen wie Entzündung oder Schmerz erzielt wird.

Im folgenden wird die Erfindung anhand zahlreicher Beispiele näher ausgeführt.

### Beispiel 1:

Der Therapeut brachte unter fakultativer, simultaner (Bildwandler, CT, Sonographie, MRI, etc.) oder nachträglicher (Röntgen, CT, MRI, Sonographie, Arthroskopie etc.) bildgebender Kontrolle eine Spritzennadel in den Gelenksraum eines Kniegelenkes und injizierte 9 ml einer Lösung von 500nM (ca. 0.003mg) Resiniferatoxin in den intrakapsulären Raum. Der Patient verspürte bereits 14 Stunden nach dem Eingriff deutliche Linderung seiner Beschwerden. Diese hielt für über 6 Monate an.

### Beispiel 2:

Der Therapeut brachte unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes und injizierte 20 ml einer Lösung von 500nM (ca. 0.006mg) Resiniferatoxin in den intrakapsulären Raum. Der Patient verspürte bereits wenige Tage nach dem Eingriff deutliche Linderung seiner Beschwerden. Diese hielt für über 6 Monate an.

### Beispiel 3:

Die injizierte Lösung entsprach derjenigen von Beispiel 1 mit dem Unterschied, dass für das zu verwendende bildgebende Verfahren 5 ml eines sichtbaren Kontrastmittels (lopamidol in einer Konzentration von 50g/100 ml) zugesetzt wurde, welches sich nach der Injektion innerhalb der Gelenkskapsel ausbreitete und so die Lage der Injektionsnadel und die Verteilung des RTX innerhalb der Kapsel dokumentierte. Die RTX enthaltende, injizierten Lösung wurde unmittelbar nach erfolgter Injektion wieder abgesogen. Sie könnte aber auch nach einer definierten substanzabhängigen Einwirkzeit oder gar nicht wieder abgesogen werden. Der Patient verspürte bereits 15 Stunden nach dem Eingriff deutliche Linderung seiner Beschwerden. Diese hielt für über 8 Monate an.

### Beispiel 4:

Der Therapeut legte einen dünnen Infusionskatheter analog zu einem Epiduralkatheter in das betroffene Gelenk ein und injizierte mit einem Perfusor eine Lösung von 1 Liter 100nM Resiniferatoxin in das betroffene Gelenk mit einer Rate von 1-10 ml/h während 12 h. Fakultativ legte er noch einen Abflusskatheter ein mit einem fakultativ definierten Abflusswiderstand (z.B. 20 mm Hg), um einen Flüssigkeitsumsatz zu erreichen. Mit dieser Methode erreichte der Therapeut eine gleichmässige Infiltration des schmerzhaften Gelenkes, ohne grosse Konzentrationsspitzen. Ausserdem konnte so die Einwirkzeit besser definiert werden.
Bei einer nachfolgenden Arthroskopie nach 1, 2, 7, 14 und 28 d konnte gezeigt werden, dass nur sehr wenig entzündliches Gewebe vorhanden war. Der Patient verspürte bereits 12 Stunden nach dem Eingriff deutliche Linderung seiner Beschwerden. Diese hielt für über 1 Jahr an.

### Beispiel 5:

Nach Implantation einer Kniegelenkprothese injizierte der Therapeut 50 ml eine Lösung von 100nM (ca. 0.001mg) Resiniferatoxin in die wieder verschlossene Gelenkskapsel. Dadurch konnten die postoperativen Schmerzen minimiert werden.

### Beispiel 6:

Nach Implantation einer Hüftgelenkprothese injizierte der Therapeut 50 ml eine Lösung von 100nM (ca. 0.001 mg) Resiniferatoxin in den periprothetischen Bereich ohne Kapsel. Dadurch konnten die postoperativen Schmerzen minimiert werden.

### Beispiel 7:

Bei einem Patienten mit schmerzhafter septischer Lockerung einer Hüfttotalendoprothese wurde eine Lösung von 5µM Resiniferatoxin (0.03mg) in die (Neo)-Kapsel um die Prothese gespritzt werden, was dazu führte, dass der Patient darauf eine dauerhafte (über ein Jahr) Linderung der Schmerzen innerhalb von wenigen (6-12) Stunden erfuhr. Ausserdem wurde die Infektion um die Prothese durch die Diffusion des RTX (welche ebenfalls antiseptisch wirkte) entlang des Prothesenschaftes und um die Pfanne stark eingedämmt und in einigen Fällen sogar komplett eliminiert werden. Fakultativ kann diese Behandlung mit systemisch verabreichten Antibiotika (z.B. mit Rifampicin 450 mg, Ciprofloxacin 750 mg) unterstützt werden.
Radiologisch konnte eine Konsolidierung der Knochensubstanz um die Prothese gezeigt werden.

### Beispiel 8:

Der Therapeut brachte unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes und injizierte 9 ml einer Lösung von 500nM (ca. 0,003mg) Resiniferatoxin gepuffert auf pH 8,5 mit einem Puffer zusammen mit physiologischer Kochsalzlösung in den intrakapsulären Raum. Der Patient verspürte bereits wenige Minuten nach dem Eingriff deutliche Linderung seiner Beschwerden. Diese hielt für über 6 Monate an.

### Beispiel 9:

Der Therapeut brachte unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes und injizierte 9 ml einer Lösung von 500nM (ca. 0,003mg) Resiniferatoxin gepuffert auf pH 6,5 mit einem Puffer zusammen mit physiologischer Kochsalzlösung in den intrakapsulären Raum. Der Patient verspürte bereits Minuten nach dem Eingriff deutliche Linderung seiner Beschwerden. Diese hielt für über 6 Monate an.

### Beispiel 10:

Der Therapeut brachte unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes und injizierte 9 ml einer Lösung von 500nM (ca. 0,003mg) Resiniferatoxin in physiologischer Ringerlösung mit 10mM Ca²⁺ in den intrakapsulären Raum. Der Patient verspürte bereits wenige Minuten nach dem Eingriff deutliche Linderung seiner Beschwerden. Diese hielt für über 6 Monate an.

### Beispiel 11:

Bei einem Patienten mit schmerzhafter Kapsulitis von Gelenken ("Frozen shoulder") wurde 9ml 100nM (ca. 0.001mg) Resiniferatoxin in physiologischer Kochsalzlösung in das Gelenk injiziert. Wiederum konnte die Verteilung der Substanz, bei Zusatz der entsprechenden Kontrastmittel bildgebend kontrolliert werden. Fakultativ wurde eine antiphlogistisch wirksame Substanz beigemischt. Wenige Minuten nach der Injektion liessen die Schmerzen dauerhaft nach, so dass der Patient mit Physiotherapie die durch die Kapsulitis verlorene Beweglichkeit wiedergewann. Bei dieser Anwendung ist manchmal lediglich eine vorübergehende Analgesie (2-3 Wochen) gewünscht, weshalb hier die Konzentration der neurotoxischen Substanz eher tief gehalten wurde

### Beispiel 12:

Bei einem Patienten mit schmerzhafter Kapsulitis von Gelenken wurde eine Lösung von 3ml 500nM (ca. 0,001 mg) Resiniferatoxin in physiologischer Kochsalzlösung in das Gelenk injiziert. Wenige Minuten nach der Injektion liessen die Schmerzen dauerhaft nach, so dass der Patient mit Physiotherapie die durch die Kapsulitis verlorene Beweglichkeit wiedergewann.

### Beispiel 13:

Der Therapeut injizierte in einen chronisch entzündeten Schleimbeutel (Bursa trochanterica) über den Trochanter major der Hüfte 5 ml einer Lösung von 500nM (ca. 0,001 mg) Resiniferatoxin gepuffert auf pH 8,5 mit einem Puffer zusammen mit physiologischer Kochsalzlösung als Lösungsmittel. Innerhalb von 60 Minuten verschwanden die Beschwerden des Patienten, der mehrere Jahre an dieser Stelle beschwerdefrei blieb.

### Beispiel 14:

Der Therapeut injizierte in eine chronisch entzündete Pleurahöhle 50ml 100nM (ca. 0,001mg) Resiniferatoxin in Glyzerin oder Ringerlactat als Lösungsmittel. Innerhalb von 60 Minuten verschwanden die Beschwerden des Patienten, der mehrere Jahre an dieser Stelle beschwerdefrei blieb.

## Patentansprüche

1. Verwendung von Resiniferatoxin (RTX) für die Herstellung eines Mittels zur Behandlung von Schmerzen bei
Arthrose
Arthritis, insbesondere Rheumatoider Arthritis und Infektiöser Arthritis
Chondrocalzinose
Ligamentären Schäden
Meniskusläsion
Knorpelschaden
Synovitis
Arthrofibrose
Morbus Sudeck
Nekrose der Gelenkanteile
Neuropathischen Gelenkschmerzen;
Behandlung von Knochenschmerzen nach Knochen-Operation durch Auftragung auf
den Knochen, z.B. nach
Beckenkammosteotomie
Hallux-Valgus Korrektur;
Behandlung von Knochenschmerzen durch Injektion in den Knochen
z.B. bei Femurkopfnekrose in den Femurkopf
in den Wirbelkörper bei Osteochondrose;
Behandlung von Schmerzen bei Gelenksteife, insbesondere
Arthrofibrose
Frozen shoulder;
Behandlung von Muskelschmerzen, durch intramuskuläre Injektion, z.B. bei
Muskelfaserriss
Muskelkater
Spastischen Erkrankungen;
Injektion in den schmerzhaften Meniskus bei Meniskusdegeneration oder Meniskusriss Behandlung von Rückenschmerzen durch Injektion in die Bandscheibe bei Bandscheibendegeneration oder Bandscheibenriss;
Injektion um einen schmerzhaften Nerven z.B. bei:
Trigeminusneuralgie
Neurinom
Morton Neurom
Phantomschmerz
Narbenneurom;
Behandlung von Zahnschmerzen intra/peridental bei:
Karies
Allen Formen von Zahnschmerz
Vor/bei/nach Zahnextraktion
Vor/bei/nach Zahnimplantat
Anwendung bei Parodontitis
Anwendung bei freiliegenden Zahnhälsen;
Injektion in die Pleurahöhle bei pleuritischen Schmerzen;
Instillation in den Darm bei Darm-Schmerzen, insbesondere Colitis Ulcerosa, M. Crohn, Analfissur, Hämorrhoiden;
Gelenkschmerzen;
Knochenschmerzen nach Osteotomie;
Schmerzen bei frozen shoulder;
Schmerzen bei Tendonitis
Schmerzen bei Myalgia;
Schmerzen bei Weichteiltumoren
Knochenschmerzen oder
Knochengelenksschmerzen,
**dadurch gekennzeichnet, dass**
A) das Mittel zusätzlich ein Kalziumsalz enthält;
B) die Kalziumionenkonzentration grösser als 2 mMol beträgt;
C) das Mittel in einer Pufferlösung mit einem pH-Wert höher als 7,6, vorzugsweise höher als 8,5 , aufgelöst ist; und
D) das Mittel kein Lokalanästhetikum enthält.

2. Verwendung von Resiniferatoxin (RTX) für die Herstellung eines Mittels zur Behandlung von
a) lokalen Wundschmerzen nach OP in Form einer Spüllösung zur intraoperativen Anwendung bei einer offenen oder arthroskopischen oder endoskopischen Operation oder Liposuction;
b) lokale Behandlung von Gelenkschmerzen durch intraartikuläre Injektion bei
Chondrocalzinose
Ligamentären Schäden
Meniskusläsion
Knorpelschaden
Synovitis
Arthrofibrose
Morbus Sudeck
Nekrose der Gelenkanteile
Neuropathischen Gelenkschmerzen
c) lokale Behandlung von Knochenschmerzen nach Knochen-Operation durch Auftragung auf den Knochen, z.B. nach
Beckenkammosteotomie
Hallux-Valgus Korrektur
d) Behandlung von Knochenschmerzen durch Injektion in den Knochen, insbesondere bei Femurkopfnekrose in den Femurkopf, in den Wirbelkörper bei Osteochondrose
e) Lokale Behandlung von Schmerzen bei_Gelenksteife, insbesondere bei Arthrofibrose oder Frozen shoulder;
f) Lokale Behandlung von Muskelschmerzen, durch intramuskuläre Injektion, insbesondere bei Muskelfaserriss, Muskelkater, oder spastischen Erkrankungen;
g) Lokale Injektion in den schmerzhaften Meniskus bei Meniskusdegeneration oder Meniskusriss;
h) Behandlung von Rückenschmerzen durch Injektion in die Bandscheibe bei Bandscheibendegeneration oder Bandscheibenriss;
i) Injektion um einen schmerzhaften Nerven, insbesondere bei Trigeminusneuralgie, Neurinom, Morton Neurom, Phantomschmerz, Narbenneurom;
k) Behandlung von Zahnschmerzen durch lokale Anwendung *intra*/*peridental,* insbesondere bei Karies, allen Formen von Zahnschmerz, Vor/bei/nach Zahnextraktion, Vor/bei/nach Zahnimplantat, Lokale Anwendung bei Parodontitis, Lokale Anwendung bei freiliegenden Zahnhälsen;
k) Injektion in die Pleurahöhle bei pleuritischen Schmerzen;
l) Instillation in den Darm bei Darmschmerzen, insbesondere bei Colitis Ulcerosa, M.Crohn, Analfissur;
**dadurch gekennzeichnet, dass**
A) das Mittel zusätzlich ein Kalziumsalz enthält;
B) die Kalziumionenkonzentration grösser als 2 mMol beträgt;
C) das Mittel in einer Pufferlösung mit einem pH-Wert höher als 7,6, vorzugsweise höher als 8,5 , aufgelöst ist;
D) keine weiteren pharmakologisch wirksamen Substanzen mitverwendet werden; und
E) das Mittel einen Permeationsförderer enthält.

3. Verwendung von Resiniferatoxin (RTX) für die Herstellung eines Mittels zur lokalen Behandlung von
- postoperativen Schmerzzustände
- Schmerzen bei Arthritis
- für lokale Wundschmerzen nach OP in Form einer Spüllösung zur intraoperativen Anwendung bei einer offenen oder arthroskopischen oder endoskopischen Operation, inklusive Liposuction;
Gelenkschmerzen;
Knochenschmerzen nach Osteotomie;
- Schmerzen bei frozen shoulder
- für lokale Wundschmerzen nach OP in Form einer Spüllösung zur intraoperativen Anwendung bei einer offenen oder arthroskopischen oder endoskopischen Operation, inklusive Liposuction;
- Lokale Behandlung von Gelenkschmerzen durch intraarticuläre Injektion bei
Arthrose
Rheumatoider Arthritis
Infektiöser Arthritis
Chondrocalzinose
Ligamentären Schäden
Meniskusläsion
Knorpelschaden
Synovitis
Arthrofibrose
Morbus Sudeck
Nekrose der Gelenkanteile
Neuropathischen Gelenkschmerzen;
Lokale Behandlung von Knochenschmerzen nach Knochen-Operation durch Auftragung
auf den Knochen, z.B. nach
Beckenkammosteotomie
Hallux-Valgus Korrektur;
Behandlung von Knochenschmerzen durch Injektion in den Knochen
z.B. bei Femurkopfnekrose in den Femurkopf
in den Wirbelkörper bei Osteochondrose;
Lokale Behandlung von Schmerzen bei Gelenksteife, vorzugsweise Arthrofibrose oder Frozen shoulder;
Lokale Behandlung von Muskelschmerzen, durch intramuskuläre Injektion, vorzugsweise bei Muskelfaserriss, Muskelkater oder spastischen Erkrankungen; Lokale Injektion in den schmerzhaften Meniskus bei Meniskusdegeneration oder Meniskusriss;
Behandlung von Rückenschmerzen durch Injektion in die Bandscheibe bei Bandscheibendegeneration oder Bandscheibenriss;
Injektion um einen schmerzhaften Nerven, insbesondere bei Trigeminusneuralgie, Neurinom, Morton Neurom, Phantomschmerz und Narbenneurom; Behandlung von Zahnschmerzen durch lokale Anwendung intra/peridental bei:
Karies
allen Formen von Zahnschmerz
vor/bei/nach Zahnextraktion
Vor/bei/nach Zahnimplantat
Lokale Anwendung bei Parodontitis
Lokale Anwendung bei freiliegenden Zahnhälsen;.
Injektion in die Pleurahöhle bei pleuritischen Schmerzen;
Instillation in den Darm bei Darmschmerzen, insbesondere Colitis Ulcerosa, M.Crohn und Analfissur;
**dadurch gekennzeichnet, dass**
A) das Mittel zusätzlich ein Kalziumsalz enthält;
B) die Kalziumionenkonzentration grösser als 2 mMol beträgt;
C) das Mittel in einer Pufferlösung mit einem pH-Wert höher als 7,6, vorzugsweise höher als 8,5 , aufgelöst ist; und
D) das Mittel einen Permeationsförderer enthält.

4. Verwendung nach Anspruch 3, **dadurch gekennzeichnet, dass** das Mittel kein Lokalanästhetikum enthält.

5. Verwendung nach einem der Ansprüche 1 bis 4, **dadurch gekennzeichnet, dass** die Konzentration an RTX zwischen 100 nM bis 10µM liegt.

6. Verwendung nach Anspruch 5, **dadurch gekennzeichnet, dass** die Konzentration an RTX zwischen 500nM bis 1µM liegt.

7. Verwendung nach einem der Ansprüche 1 bis 6, **dadurch gekennzeichnet, dass** das Mittel keinen Alkohol, insbesondere kein Ethanol enthält.

8. Verwendung nach einem der Ansprüche 1 bis 7, **dadurch gekennzeichnet, dass** das Mittel zusätzlich ein Röntgenkontrastmittel enthält, vorzugsweise gadoliniumhaltige, jodhaltige oder bariumhaltige Substanzen.

9. Verwendung nach einem der Ansprüche 1 bis 8, **dadurch gekennzeichnet, dass** das Mittel zusätzlich Glyzerin enthält, vorzugsweise in einer Konzentration von 10 bis 95 Gew.-Prozent.

10. Verwendung nach einem der Ansprüche 1 oder 3 bis 9, **dadurch gekennzeichnet, dass** das Mittel zusätzlich ein Steroid enthält.

11. Verwendung nach einem der Ansprüche 1 oder 3 bis 10, **dadurch gekennzeichnet, dass** das Mittel zusätzlich einen Vasokonstriktor enthält, vorzugsweise Adrenalin, Noradrenalin, Phenylephrin oder Ornipressin.

12. Verwendung nach einem der Ansprüche 1 bis 11, **dadurch gekennzeichnet, dass** das Mittel in einem körperverträglichen Lösungsmittel gelöst ist, vorzugsweise in Glyzerin, lophendylat oder Propylenglykol.

13. Verwendung nach einem der Ansprüche 1 bis 12, **dadurch gekennzeichnet, dass** das Mittel zur Denervation oder Neurolyse in degenerativ erkrankten Gelenken verwendet werden.

14. Verwendung nach einem der Ansprüche 1 bis 13, **dadurch gekennzeichnet, dass** das Mittel in einer Trägerflüssigkeit (Carrier), einem pharmakologisch akzeptablem Vehikel, insbesondere aus der Gruppe Natriumchlorid-Injektionslösung, Ringiers Injektionslösung, isotonische Dextrose, steriles Wasser Dextroselösung, Laktierte Ringers Injektionslösung, destilliertes Wasser oder Mischungen davon. zur lokalen Injektion aufgelöst ist.

15. Verwendung nach einem der Ansprüche 1 bis 6 oder 8 bis 14, **dadurch gekennzeichnet, dass** der Permeationsförderer aus folgender Gruppe von Substanzen ausgewählt ist: Dimethylsulfoxid, Ethoxyethylendiglycol, Ethanol, Phosphatidylcholine, Propylenglycol dipelargonate (DPPG), oder glycolysierte ethoxylierte Glyceride.

16. Verwendung nach einem der Ansprüche 1 bis 15, **dadurch gekennzeichnet, dass** das Mittel zusätzlich eine Substanz enthält, welche eine verzögerte und prolongierte Freisetzung des RTX ermöglicht.

17. Verwendung nach Anspruch 16, **dadurch gekennzeichnet, dass** die Substanz Glucosaminoglycane oder Hyaluronsäure ist.

18. Verwendung nach einem der Ansprüche 1 bis 17, **dadurch gekennzeichnet, dass** das Mittel einen Permeationsförderer enthält;

19. Verwendung nach einem der Ansprüche 1 bis 18, **dadurch gekennzeichnet, dass** die Kalziumionenkonzentration grösser als 4 mMol beträgt.

20. Verwendung nach Anspruch 18 oder 19, **dadurch gekennzeichnet, dass** die im Lösungsmedium gelösten Salze und Ionen höher als physiologisch normal (z.B in Ringer Lactat Lösung) konzentriert sind.

21. Mittel zur Verwendung bei der Behandlung von Schmerzen, **dadurch gekennzeichnet, dass** Resiniferatoxin (RTX) in einem geeigneten körperverträglichen Lösungsmittel gelöst ist und vorzugsweise ein Flüssigkeitsvolumen von 0,1 bis 150 ml davon
a) lokal in die schmerzbefallene Gewebestruktur des Patienten injiziert wird; oder
b) lokal auf die Operationswunde geträufelt wird; oder
c) in den intrakapsulären Bereich lokal injiziert wird; oder
d) in den Gelenkschmierbeutel eines von Schmerzen betroffenen Gelenkes lokal injiziert wird,
**dadurch gekennzeichnet, dass**
A) das Mittel zusätzlich ein Kalziumsalz enthält;
B) die Kalziumionenkonzentration grösser als 2 mMol beträgt;
C) das Mittel in einer Pufferlösung mit einem pH-Wert höher als 7,6, vorzugsweise höher als 8,5 , aufgelöst ist; und
D) das Resiniferatoxin (RTX) in einer solchen Konzentration verwendet wird, dass eine Neurolyse auftritt.

22. Mittel zur Verwendung nach Anspruch 21, **dadurch gekennzeichnet, dass** die nociceptiven Nervenfasern durch das Resiniferatoxin (RTX) für mindestens 14 Tage, vorzugsweise mindestens 8 Wochen schmerzunempfindlich gemacht werden.

23. Mittel zur Verwendung nach Anspruch 21 oder 22, **dadurch gekennzeichnet, dass** die Konzentration des RTX zwischen ungefähr 10nanoMolar (nM) bis 100 MikroMolar (µM) liegt.

24. Mittel zur Verwendung nach einem der Ansprüche 21 - 23, **dadurch gekennzeichnet, dass** es für folgende Indikationen angewendet wird:
- für lokale Wundschmerzen nach OP in Form einer Spüllösung zur intraoperativen Anwendung bei einer offenen oder arthroskopischen oder endoskopischen Operation sowie Liposuction;
- Lokale Behandlung von Gelenkschmerzen durch intraarticuläre Injektion bei
Arthrose
Rheumatoider Arthritis
Infektiöser Arthritis
Chondrocalzinose
Ligamentären Schäden
Meniskusläsion
Knorpelschaden
Synovitis
Arthrofibrose
Morbus Sudeck
Nekrose der Gelenkanteile
Neuropathischen Gelenkschmerzen;
Lokale Behandlung von Knochenschmerzen nach Knochen-Operation durch Auftragung auf den Knochen, z.B. nach Beckenkammosteotomie oder Hallux-Valgus Korrektur;
Behandlung von Knochenschmerzen durch Injektion in den Knochen z.B. bei Femurkopfnekrose in den Femurkopf, in den Wirbelkörper bei Osteochondrose Lokale Behandlung von Schmerzen bei_Gelenksteife, vorzugsweise bei Arthrofibrose oder Frozen shoulder;
Lokale Behandlung von Muskelschmerzen, durch intramuskuläre Injektion, vorzugsweise bei Muskelfaserriss, Muskelkater oder spastischen Erkrankungen; Lokale Injektion in den schmerzhaften Meniskus bei Meniskusdegeneration oder Meniskusriss,
Behandlung von Rückenschmerzen durch Injektion in die Bandscheibe bei Bandscheibendegeneration oder Bandscheibenriss;
Injektion um einen schmerzhaften Nerven, vorzugsweise bei Trigeminusneuralgie, Neurinom, Morton Neurom, Phantomschmerz oder Narbenneurom; Behandlung von Zahnschmerzen durch lokale Anwendung intra/peridental bei:
Karies
Allen Formen von Zahnschmerz
Vor/bei/nach Zahnextraktion
Vor/bei/nach Zahnimplantat
Lokale Anwendung bei Parodontitis
Lokale Anwendung bei freiliegenden Zahnhälsen;
Injektion in die Pleurahöhle bei pleuritischen Schmerzen;
Instillation in den Darm bei Darmschmerzen, insbesondere bei Colitis Ulcerosa, M.Crohn oder Analfissur.

25. Mittel zur Verwendung nach einem der Ansprüche 21 bis 24, **dadurch gekennzeichnet, dass** das Mittel in eine synoviale Höhle eingespritzt wird, welche nicht mit Urothel ausgekleidet ist.

26. Resiniferatoxin zur Verwendung in einem Mittel zur Behandlung von
Arthrose
Arthritis, insbesondere rheumatoider Arthritis und Infektiöser Arthritis
Chondrocalzinose
Ligamentären Schäden
Meniskusläsion
Knorpelschaden
Synovitis
Arthrofibrose
Morbus Sudeck
Nekrose der Gelenkanteile
Neuropathischen Gelenkschmerzen
Behandlung von Knochenschmerzen nach Knochen-Operation durch Auftragung auf
den Knochen, z.B. nach
Beckenkammosteotomie
Hallux-Valgus Korrektur
Behandlung von Knochenschmerzen durch Injektion in den Knochen
z.B. bei Femurkopfnekrose in den Femurkopf
in den Wirbelkörper bei Osteochondrose
Behandlung von Schmerzen bei Gelenksteife, insbesondere
Arthrofibrose
Frozen shoulder
Behandlung von Muskelschmerzen, durch intramuskuläre Injektion, z.B. bei
Muskelfaserriss
Muskelkater
Spastischen Erkrankungen
Injektion in den schmerzhaften Meniskus bei Meniskusdegeneration oder Meniskusriss Behandlung von Rückenschmerzen durch Injektion in die Bandscheibe bei Bandscheibendegeneration oder Bandscheibenriss;
Injektion um einen schmerzhaften Nerven z.B. bei:
Trigeminusneuralgie
Neurinom
Morton Neurom
Phantomschmerz
Narbenneurom;
Behandlung von Zahnschmerzen intra/peridental bei:
Karies
Allen Formen von Zahnschmerz
Vor/bei/nach Zahnextraktion
Vor/bei/nach Zahnimplantat
Anwendung bei Parodontitis
Anwendung bei freiliegenden Zahnhälsen;
Injektion in die Pleurahöhle bei pleuritischen Schmerzen;
Instillation in den Darm bei Darmschmerzen, insbesondere Colitis Ulcerosa, M. Crohn, Analfissur, Hämorrhoiden;
Gelenkschmerzen;
Knochenschmerzen nach Osteotomie;
frozen shoulder;
Tendonitis;
Myalgia;
Schmerzen bei Weichteiltumoren;
Knochenschmerzen oder
Knochengelenksschmerzen;
**dadurch gekennzeichnet, dass**
A) das Mittel zusätzlich ein Kalziumsalz enthält;
B) die Kalziumionenkonzentration grösser als 2 mMol beträgt;
C) das Mittel in einer Pufferlösung mit einem pH-Wert höher als 7,6, vorzugsweise höher als 8,5 , aufgelöst ist; und
D) das Mittel kein Lokalanästhetikum enthält.

## Claims

1. Use of resiniferatoxin (RTX) for the preparation of an agent for the treatment of pain in the case of
arthrosis
arthritis, especially rheumatoid arthritis and infectious arthritis,
chondrocalcinosis
ligamentary injuries
meniscus lesions
cartilage damage
synovitis
arthrofibrosis
Sudeck's atrophy
necrosis of sections of a joint
neuropathic joint pains
treatment of bone pain after bone surgery by application on the bones, for example,
after
pelvic osteotomy
Hallux valgus correction;
treatment of bone pain by injection into the bone
for example, in the case of a necrosis in the head of the femur
in the vertebrae in the case of osteochondrosis
treatment of joint stiffness pain, especially
arthrofibrosis
frozen shoulder;
treatment of muscle pain by intramuscular injection, for example, in the case of
a muscle fiber tear
muscular soreness
spastic diseases;
injection into the painful meniscus in the case of a degeneration of or tear in the meniscus
treatment of back pain by injection into the intervertebral disc in the case of a degeneration of or a tear in the intervertebral disc
injection about a painful nerve, for example, in the case of:
trigeminal neuralgia
neurinoma
morton's neuroma
phantom pain
neuroma
treatment of intradental and/or peridental toothache in the case of:
caries
all forms of toothache
before and/or during and/or after a tooth extraction
before and/or during and/or after a dental implant
use in the case of parodontitis
use in the case of an exposed neck of a tooth
injection into the pleural cavity in the case of pleuritic pain
instillation into the intestines in the case of intestinal pain, especially ulcerating colitis, Crohn's
disease,
anal fissure, hemorrhoids;
joint pain
bone pain after an osteotomy;
frozen shoulder pain
tendonitis pain
myalgia pain
soft tissue tumor pain
bone pain or
bone joint pain
**characterized in that**
A) the agent additionally contains a calcium salt;
B) the calcium ion concentration exceeds 2 mmoles;
C) the agent is dissolved in a buffer solution with a pH of more than 7.6 and preferably of more than 8.5 and
D) the agent does not contain a local anesthetic.

2. Use of resiniferatoxin (RTX) for the preparation of an agent for the treatment of
a) local wound pain after surgery in the form of a rinsing solution for intraoperative application in the case of open or arthroscopic or endoscopic surgery or of liposuction;
b) Local treatment of joint pain by intra-articular injection in the case of
chondrocalcinosis
ligamentary injuries
meniscus lesion
cartilage damage
synovitis
arthrofibrosis
Sudeck's atrophy
Necrosis of sections of a joint
neuropathic joint pains
c) local treatment of bone pain after bone surgery by
application on the bone, for example after
Hallux valgus correction,
d) treatment of bone pain by injection into the bone, especially in the case of necrosis in the head of the femur and/or in the vertebrae in the case of osteochondrosis
e) local treatment of pain in the case of joint stiffness, especially in the case of arthrofibrosis or frozen shoulder;
f) local treatment of muscle pain by intramuscular injection, especially in the case of a muscle fiber tear, muscular soreness or spastic diseases;
g) local injection into the painful meniscus in the case of a degeneration of or tear in the meniscus
h) treatment of back pain by injection into the intervertebral disc in the case of a degeneration of or a tear in the intervertebral disc
i) injection about a painful nerve, especially in the case of trigeminal neuralgia, neurinoma, morton's neuroma, phantom pain, neuroma;
k) treatment of a toothache by a topical intradental or peridental application, especially in the case of caries, all forms of toothache, before and/or during and/or after a tooth extraction, before and/or during and/or after a dental implant, topical application in the case of parodontitis, a topical application in the case of an exposed neck of a tooth;
l) injection into the pleural cavity in the case of pleuritic pain;
m) instillation into the intestines in the case of intestinal pain, especially in the case of ulcerating colitis, Crohn's disease, anal fissures;
**characterized in that**
A) the agent additionally contains a calcium salt;
B) the calcium ion concentration exceeds 2 mmoles;
C) the agent is dissolved in a buffer solution with a pH higher than 7.6 and preferably higher than 8.5;
D) no other pharmacologically active substances are used and
E) the agent contains a permeation promoter.

3. Use of resiniferatoxin (RTX) for the preparation of an agent for the treatment of
- postoperative pain conditions
- arthritic pain
- for local wound pain after surgery in the form of a rinsing solution for intraoperative application in the case of open or arthroscopic or endoscopic surgery or of liposuction; joint pain;
bone pain after an osteotomy;
- frozen shoulder pain
- for local wound pain after surgery in the form of a rinsing solution for intraoperative application in the case of open or arthroscopic or endoscopic surgery or of liposuction;
- local treatment of joint pain by intra-articular injection in the case of
arthrosis
rheumatoid arthritis
infectious arthritis
chondrocalcinosis
ligamentary injuries
meniscus lesion
cartilage damage
synovitis
arthrofibrosis
Sudeck's atrophy
necrosis of sections of a joint
neuropathic joint pain
topical treatment of bone pain after bone surgery by application
on the bone, for example, after
pelvic osteotomy
Hallux valgus correction,
treatment of bone pain by injection into the bone
for example, in the case of a necrosis in the head of the femur
in the vertebrae in the case of osteochondrosis
local treatment of joint stiffness pain, preferably arthrofibrosis or frozen shoulder;
local treatment of muscle pain by intramuscular injection, preferably in the case of a muscle fiber tear, muscle soreness or spastic diseases;
local injection into the painful meniscus in the case of a degeneration of or a tear in the meniscus
treatment of back pain by injection into the intervertebral disc in the case of a degeneration of or a tear in the intervertebral disc;
injection about a painful nerve, especially in the case of trigeminal neuralgia, neurinoma, morton's neuroma, phantom pain and neuroma;
treatment of a toothache by topical intradental and/or peridental application in the case of
caries
all forms of toothache
before and/or during and/or after a tooth extraction
before and/or during and/or after a dental implant
topical use in the case of parodontitis
use in the case of an exposed neck of a tooth
injection into the pleural cavity in the case of pleuritic pain;
instillation into the intestines in the case of intestinal pain, especially in the case of ulcerating colitis, Crohn's disease and anal fissures,
**characterized in that**
A) the agent additionally contains a calcium salt;
B) the calcium concentration exceeds 2 mmole;
C) the agent is dissolved in a buffer solution with a pH higher than 7.6 and preferably higher than 8.5; and
D) the agent contains a permeation promoter.

4. The use of claim 3, **characterized in that** the agent does not contain a local anesthetic.

5. The use of one of the claims 1 to 4, **characterized in that** the concentration of RTX is between 100 nM and 10 pM.

6. The use of claim 5, **characterized in that** the concentration of RTX is between 500 nM and 1 pM.

7. The use of one of the claims 1 to 6, **characterized in that** the agent does not contain any alcohol and especially not any ethanol.

8. The use of one of the claims 1 to 7, **characterized in that** the agent additionally contains an x-ray contrast medium, preferably substances containing gadolinium, iodine or barium.

9. The use of one of the claims 1 to 8, **characterized in that** the agent additionally contains glycerin, preferably in a concentration of 10 to 95% by weight.

10. The use of one of the claims 1 or 3 to 9, **characterized in that** the agent additionally contains a steroid.

11. The use of one of the claims 1 or 3 to 10, **characterized in that** the agent additionally contains a vasoconstrictor, preferably adrenaline, noradrenaline, phenylephrine or omipressin.

12. The use of one of the claims 1 to 11, **characterized in that** the agent is dissolved in a bio-compatible solvent, preferably in glycerin, iophendylate or propylene glycol.

13. The use of one of the claims 1 to 12, **characterized in that** the agent for the denervation or neurolysis is used in the degeneratively diseased joints.

14. The use of one of the claims 1 to 13, **characterized in that** the agent is dissolved in a carrier liquid, a pharmacologically acceptable vehicle, especially from the group comprising a sodium chloride injection solution, Ringer's injection solution, isotonic dextrose, sterile water dextrose solution, lactated Ringer's injection solution, distilled water or mixtures thereof for the local injection.

15. The use of one of the claims 1 to 6 or 8 to 14, **characterized in that** the permeation promoter is selected from the following group of substances: dimethyl sulfoxide, ethoxyethylene diglycol, ethanol, phosphatidylcholine, propylene glycol dipelargonate (DPPG) or glycolysed ethoxylated glycerides.

16. The use of one of the claims 1 to 15, **characterized in that** the agent additionally contains a substance, which makes a retarded and prolonged release of the RTX possible.

17. The use of claim 16, **characterized in that** the substance is glucosamineglycans or hyaluronic acid.

18. The use of one of the claims 1 to 17, **characterized in that** the agent contains a permeation promoter.

19. The use of one of the claims 1 to 18, **characterized in that** the calcium ion concentration exceeds 4 mmoles.

20. The use of claim 18 or claim 19, **characterized in that** the concentration of the salts and ions dissolved in the solution medium is higher than the physiologically normal concentration for example, in ringer lactate solution.

21. An agent for use in the treatment of pain, **characterized in that** resiniferatoxin (RTX) is dissolved in a suitable bio-compatible solvent and preferably a liquid volume of 0.1 to 150 mL thereof is
a) injected locally into the pain-affected tissue structure of the patient; or
b) is added dropwise, locally on the surgical wound; or
c) is injected locally into the intracapsular region; or
d) is injected locally into the synovial pouch of a joint affected by pain
**characterized in that**
A) the agent additionally contains a calcium salt;
B) the calcium ion concentration exceeds 2 mmoles;
C) the agent is dissolved in a buffer solution with a pH of more than 7.6, preferably of more than 8.5 and
D) the resiniferatoxin (RTX) is used in such a concentration, that neurolysis occurs.

22. The agent for the use of claim 21, **characterized in that** the nociceptive nerve fibers are made insensitive to pain by resiniferatoxin (RTX) for at least 14 days and preferably for at least 8 weeks.

23. The agent for use of claim 21 or claim 22, **characterized in that** the concentration of RTX is between 10 nanomolar (nM) and 10 micromolar (pM).

24. The agent for use of one of the claims 21 - 23, **characterized in that** it is employed for the following indications:
- for local wound pain after surgery in the form of a rinsing solution for intraoperative application in the case of open or arthroscopic or endoscopic surgery or of liposuction;
- local treatment of joint pain by intra-articular injection in the case of arthrosis
rheumatoid arthritis
infectious arthritis
chondrocalcinosis
ligamentary injuries
meniscus lesions
cartilage damage
synovitis
arthrofibrosis
Sudeck's atrophy
necrosis of sections of a joint
neuropathic joint pains
topical treatment of bone pain after bone surgery by application on the bones, for example, after pelvic osteotomy or Hallux valgus correction;
treatment of bone pain by injection into the bone, for example, in the case of a necrosis in the head of the femur, into the vertebrae in the case of osteochondrosis, topical treatment of pain associated with joint stiffness, preferably in the case of arthrofibrosis or frozen shoulder;
topical treatment of muscle pain by intramuscular injection, preferably in the case of a muscle fiber tear, muscle soreness or spastic diseases;
local injection into the painful meniscus in the case of a degeneration of or a tear in the meniscus,
treatment of back pain by injection into the intervertebral disc in the case of a degeneration of or a tear in the intervertebral disc;
injection about a painful nerve, especially in the case of trigeminal neuralgia, neurinoma, morton's neuroma, phantom pain or neuroma;
treatment of a toothache by topical intradental and/or peridental application in the case of
caries
all forms of toothache
before and/or during and/or after a tooth extraction
before and/or during and/or after a dental implant
topical use in the case of parodontitis
topical use in the case of an exposed neck of a tooth
injection into the pleural cavity in the case of pleuritic pain;
instillation into the intestines in the case of intestinal pain, especially in the case of ulcerating colitis, Crohn's disease or anal fissures.

25. The agent for a use of one of the claims 21 to 24, **characterized in that** the agent is injected into a synovial cavity, which is not lined with urothelium.

26. Resiniferatoxin for use in an agent for the treatment of
arthrosis
arthritis, especially rheumatoid arthritis and infectious arthritis,
chondrocalcinosis
ligamentary injuries
meniscus lesion
cartilage damage
synovitis
arthrofibrosis
Sudeck's atrophy
Necrosis of sections of a joint
neuropathic joint pains
treatment of bone pain after bone surgery by application on the bones, for example, after
pelvic osteotomy
Hallux valgus correction
treatment of bone pain by injection into the bone
for example, in the case of a necrosis in the head of the femur
in the vertebrae in the case of osteochondrosis
treatment of joint stiffness pain, especially
arthrofibrosis
frozen shoulder;
treatment of muscle pain by intramuscular injection, for example, in the case of
a muscle fiber tear
muscular soreness
spastic diseases;
injection into the painful meniscus in the case of a degeneration of or tear in the meniscus
treatment of back pain by injection into the intervertebral disc in the case of
a degeneration of or a tear in the intervertebral disc;
injection about a painful nerve, for example, in the case of:
trigeminal neuralgia
morton's neurinoma
neurinoma
phantom pain
neuroma
treatment of intradental and/or peridental toothache in the case of:
caries
all forms of toothache
before and/or during and/or after a tooth extraction
before and/or during and/or after a dental implant
use in the case of parodontitis
use in the case of an exposed neck of a tooth
injection into the pleural cavity in the case of pleuritic pain;
instillation into the intestines in the case of intestinal pain, especially in the case of ulcerating colitis, Crohn's disease, anal fissures and/or hemorrhoids;
joint pain;
bone pain after an osteotomy;
frozen shoulder;
tendonitis;
myalgia;
soft tissue tumor pain
bone pain or
bone joint pain
**characterized in that**
A) the agent additionally contains a calcium salt;
B) the calcium ion concentration exceeds 2 mmoles;
C) the agent is dissolved in a buffer solution with a pH higher than 7.6 and preferably higher than 8.5; and
D) the agent does not contain a local anesthetic.

## Revendications

1. Utilisation de la résinifératoxine (RTX) pour la production d'un agent destiné au traitement des douleurs en cas de
arthrose
arthrite, en particulier polyarthrite rhumatoïde et arthrite infectieuse chondrocalcinose
lésions ligamentaires
lésion du ménisque
lésion cartilagineuse
synovite
fibrose articulaire
maladie de Sudeck
nécrose des parties articulaires
douleurs articulaires neuropathiques;
traitement de douleurs osseuses après chirurgie osseuse par application sur l'os, par ex. après
une ostéotomie de la crête iliaque
une correction d'un hallux valgus;
traitement de douleurs osseuses par injection dans l'os
par ex. dans la tête fémorale en cas de nécrose de la tête fémorale dans le corps vertébral en cas d'ostéochondrose ;
traitement de douleurs en cas de raideur articulaire, en particulier
fibrose articulaire
épaule gelée;
traitement de douleurs musculaires, par injection intramusculaire, par exemple en cas de
déchirure musculaire
courbatures
affections spastiques;
injection dans le ménisque douloureux en cas de dégénérescence du ménisque ou de rupture du ménisque
traitement de douleurs dorsales par injection dans le disque intervertébral en cas de dégénérescence du disque intervertébral ou de rupture du disque intervertébral ;
injection autour d'un nerf douloureux, par ex. en cas de
névralgie du trijumeau
neurinome
névrome de Morton
douleur fantôme
névrome cicatriciel ;
traitement de douleurs dentaires intra/péridentale en cas de :
carie
toutes formes de douleur dentaire
avant/pendant/après une extraction dentaire
avant/pendant/après une pose d'implant dentaire
utilisation en cas de parodontite
utilisation en cas de collets dentaires dénudés ;
injection dans la cavité pleurale en cas de douleurs pleurétiques ; instillation dans l'intestin en cas de douleurs intestinales, en particulier colite ulcéreuse, maladie de Crohn, fissure anale, hémorroïdes ;
douleurs articulaires ;
douleurs osseuses après une ostéotomie ;
douleurs en cas de épaule gelée ;
douleurs en cas de tendinite
douleurs en cas de myalgie ;
douleurs en cas de tumeurs de tissus mous
douleurs osseuses ou
douleurs ostéo-articulaires,
**caractérisée en ce que**
A)l'agent contient en outre un sel de calcium ;
B) la concentration en ions calcium est supérieure à 2 mmol ;
C) l'agent est dissous dans une solution tampon ayant un pH supérieur à 7,6, de préférence supérieur à 8,5 ; et
D) l'agent ne contient pas d'anesthésique local.

2. Utilisation de la résinifératoxine (RTX) pour la production d'un agent pour le traitement
a) des douleurs de plaies locales post-opératoires sous forme de solution de lavage pour l'application intra-opératoire en cas d'opération ouverte ou arthroscopique ou endoscopique ou de liposuccion ;
b) traitement local de douleurs articulaires par injection intra-articulaire en cas de
chondrocalcinose
lésions ligamentaires
lésion du ménisque
lésion cartilagineuse
synovite
fibrose articulaire
maladie de Sudeck
nécrose des parties articulaires
douleurs articulaires neuropathiques
c) traitement local de douleurs osseuses après chirurgie osseuse par application sur l'os, par ex. après
une ostéotomie de la crête iliaque
une correction d'un hallux valgus
d) traitement de douleurs osseuses par injection dans l'os, en particulier dans la tête fémorale en cas de nécrose de la tête fémorale, dans le corps vertébral en cas d'ostéochondrose
e) traitement local de douleurs en cas de raideur articulaire, en particulier en cas
de fibrose articulaire ou d'épaule gelée ;
f) traitement local de douleurs musculaires, par injection intramusculaire, par exemple en cas d'une déchirure musculaire, de courbatures ou d'affections spastiques ;
g) injection locale dans le ménisque douloureux en cas de dégénérescence ou de rupture du ménisque ;
h) traitement de douleurs dorsales par injection dans le disque intervertébral en cas de dégénérescence du disque intervertébral ou de rupture du disque intervertébral ;
i) injection autour d'un nerf douloureux, en particulier en cas de névralgie du trijumeau, neurinome, névrome de Morton, douleur fantôme, névrome cicatriciel ;
k) traitement *intra*/*péridentale* de douleurs dentaires par utilisation locale, en particulier en cas de carie, de toutes formes de douleur dentaire, avant/pendant/après une extraction dentaire, avant/pendant/après une pose d'implant dentaire, utilisation locale en cas de parodontite, utilisation locale en cas de collets dentaires dénudés ;
k) injection dans la cavité pleurale en cas de douleurs pleurétiques ;
l) instillation dans l'intestin en cas de douleurs intestinales, en particulier en cas de colite ulcéreuse, maladie de Crohn, fissure anale ;
**caractérisée en ce que**
A) l'agent contient en plus un sel de calcium ;
B) la concentration en ions calcium est supérieure à 2 mmol ;
C) l'agent est dissous dans une solution tampon ayant un pH supérieur à 7,6, de préférence supérieur à 8,5 ; D) aucune autre substance pharmacologiquement active n'est utilisée conjointement, et
E) l'agent contient un amplificateur de perméation.

3. Utilisation de la résinifératoxine (RTX) pour la production d'un agent destiné au traitement local
- d'états douloureux postopératoires
- de douleurs en cas d'arthrite
- pour des douleurs de plaies locales post-opératoires sous forme de solution de lavage pour l'application intra-opératoire en cas d'opération ouverte ou arthroscopique ou endoscopique, liposuccion comprise ; douleurs articulaires ;
douleurs osseuses après une ostéotomie ;
- douleurs en cas d'épaule gelée
- pour des douleurs de plaies locales post-opératoires, sous forme de solution de lavage pour l'application intra-opératoire en cas d'opération ouverte ou arthroscopique ou endoscopique, liposuccion comprise ;
- traitement local de douleurs articulaires par injection intra-articulaire en cas de
arthrose
polyarthrite rhumatoïde
arthrite infectieuse
chondrocalcinose
lésions ligamentaires
lésion du ménisque
lésion cartilagineuse
synovite
fibrose articulaire
maladie de Sudeck
nécrose des parties articulaires
douleurs articulaires neuropathiques ;
traitement local de douleurs osseuses après chirurgie osseuse par application sur l'os, par ex. après
une ostéotomie de la crête iliaque
une correction d'un hallux valgus;
traitement de douleurs osseuses par injection dans l'os
par ex. dans la tête fémorale en cas de nécrose de la tête fémorale dans le corps vertébral en cas d'ostéochondrose ;
traitement local de douleurs en cas de raideur articulaire, de préférence fibrose articulaire ou épaule gelée ;
traitement local de douleurs musculaires, par injection intramusculaire, de préférence en cas d'une déchirure musculaire, de courbatures ou d'affections spastiques ;
injection locale dans le ménisque douloureux en cas de dégénérescence du ménisque ou de rupture du ménisque ;
traitement de douleurs dorsales par injection dans le disque intervertébral en cas de dégénérescence du disque intervertébral ou de rupture du disque intervertébral ;
injection autour d'un nerf douloureux, en particulier en cas de névralgie du trijumeau, neurinome, névrome de Morton, douleur fantôme et névrome cicatriciel ;
traitement *intra*/*péridentale* de douleurs dentaires par utilisation locale, en cas de
carie
de toutes formes de douleur dentaire avant/pendant/après une extraction dentaire avant/pendant/après une pose d'implant dentaire utilisation locale en cas de parodontite utilisation locale en cas de collets dentaires dénudés ;
injection dans la cavité pleurale en cas de douleurs pleurétiques ; instillation dans l'intestin en cas de douleurs intestinales, en particulier en cas de colite ulcéreuse, maladie de Crohn et de fissure anale ;
**caractérisée en ce que**
A)l'agent contient en outre un sel de calcium ;
B) la concentration en ions calcium est supérieure à 2 mmol ;
C) l'agent est dissous dans une solution tampon ayant un pH supérieur à 7,6, de préférence supérieur à 8,5 ; et
D) l'agent contient un amplificateur de perméation.

4. Utilisation selon la revendication 3, **caractérisée en ce que** l'agent ne contient pas d'anesthésique local.

5. Utilisation selon une des revendications 1 à 4, **caractérisée en ce que** la concentration en RTX est comprise entre 100 nM et 10 µM.

6. Utilisation selon la revendication 5, **caractérisée en ce que** la concentration en RTX est comprise entre 500 nM et 1 µM.

7. Utilisation selon une des revendications 1 à 6, **caractérisée en ce que** l'agent ne contient pas d'alcool, en particulier pas d'éthanol.

8. Utilisation selon une des revendications 1 à 7, **caractérisée en ce que** l'agent contient en outre un agent de contraste radiologique, de préférence des substances contenant du gadolinium, de l'iode ou du baryum.

9. Utilisation selon une des revendications 1 à 8, **caractérisée en ce que** l'agent contient en outre de la glycérine, de préférence à une concentration de 10 à 95 pourcent en poids.

10. Utilisation selon une des revendications 1 ou 3 à 9, **caractérisée en ce que** l'agent contient en plus un stéroïde.

11. Utilisation selon une des revendications 1 ou 3 à 10, **caractérisée en ce que** l'agent contient en plus un vasoconstricteur, de préférence de l'adrénaline, de la noradrénaline, de la phénylephrine ou de l'ornipressine.

12. Utilisation selon une des revendications 1 à 11, **caractérisée en ce que** l'agent est dissous dans un solvant toléré par le corps, de préférence dans de la glycérine, de l'iophendylate ou du propylène glycol.

13. Utilisation selon une des revendications 1 à 12, **caractérisée en ce que** l'agent est utilisé pour la dénervation ou la neurolyse dans des articulations affectées par une dégénérescence.

14. Utilisation selon une des revendications 1 à 13, **caractérisée en ce que** l'agent est dissous dans un liquide porteur (carrier), un véhicule pharmacologiquement acceptable, en particulier parmi le groupe de solution injectable de chlorure de sodium, solution injectable de Ringer, dextrose isotonique, eau stérile solution de dextrose, solution injectable de Ringer lactate, eau distillée ou mélanges de ceux-ci pour l'injection locale.

15. Utilisation selon une des revendications 1 à 6 ou 8 à 14, **caractérisée en ce que** l'amplificateur de perméation est sélectionné parmi le groupe suivant de substances : diméthylsulfoxyde, éthoxyéthylènediglycol, éthanol, phosphatidylcholine, dipélargonate de propylène glycol (DPPG) ou glycérides glycolysés éthoxylés.

16. Utilisation selon une des revendications 1 à 15, **caractérisée en ce que** l'agent contient en plus une substance qui permet une libération retardée et prolongée de la RTX.

17. Utilisation selon la revendication 16, **caractérisée en ce que** la substance est un glycosaminoglycane ou l'acide hyaluronique.

18. Utilisation selon une des revendications 1 à 17, **caractérisée en ce que** l'agent contient un amplificateur de perméation.

19. Utilisation selon une des revendications 1 à 18, **caractérisée en ce que** la concentration en ions calcium est supérieure à 4 mmol.

20. Utilisation selon la revendication 18 ou 19, **caractérisée en ce que** les sels et les ions dissous dans le milieu de dissolution sont plus fortement concentrés que ce qui est physiologiquement normal (par ex. dans une solution de Ringer lactate).

21. Agent pour l'utilisation lors du traitement de douleurs, **caractérisé en ce que** la résinifératoxine (RTX) est dissoute dans un solvant approprié toléré par le corps, et **en ce que** de préférence un volume de liquide de 0,1 à 150 ml de celui-ci
a) est injecté localement dans la structure tissulaire du patient affectée par la douleur ; ou
b) est instillé localement sur la plaie opératoire ; ou
c) est injecté localement dans la zone intracapsulaire ; ou
d) est injecté localement dans la poche de liquide synovial d'une articulation affectée par des douleurs,
**caractérisé en ce que**
A) l'agent contient en plus un sel de calcium ;
B) la concentration en ions calcium est supérieure à 2 mmol ;
C) l'agent est dissous dans une solution tampon ayant un pH supérieur à 7,6, de préférence supérieur à 8,5 ; et
D) la résinifératoxine (RTX) est utilisée à une concentration telle qu'il survient une neurolyse.

22. Agent pour l'utilisation selon la revendication 21, **caractérisé en ce que** les fibres nerveuses nociceptives sont rendues insensibles à la douleur par la résinifératoxine (RTX) pour au moins 14 jours, de préférence pour au moins 8 semaines.

23. Agent pour l'utilisation selon la revendication 21 ou 22, **caractérisé en ce que** la concentration de la RTX est comprise entre environ 10 nanomolaires (nM) à 100 micromolaires (µM).

24. Agent pour l'utilisation selon une des revendications 21 - 23, **caractérisé en ce qu'**il est utilisé pour les indications suivantes :
- pour des douleurs de plaies locales post-opératoires, sous forme de solution de lavage pour l'applicationintra-opératoire en cas d'opération ouverte ou arthroscopique ou endoscopique ainsi que de liposuccion ;
- traitement local de douleurs articulaires par injection intra-articulaire en cas de
arthrose
polyarthrite rhumatoïde
arthrite infectieuse
chondrocalcinose
lésions ligamentaires
lésion du ménisque
lésion cartilagineuse
synovite
fibrose articulaire
maladie de Sudeck
nécrose des parties articulaires
douleurs articulaires neuropathiques ;
traitement local de douleurs osseuses après chirurgie osseuse par application sur l'os, par ex. après une ostéotomie de la crête iliaque ou une correction d'un hallux valgus ;
traitement de douleurs osseuses par injection dans l'os par ex. dans la tête fémorale en cas de nécrose de la tête fémorale, dans le corps vertébral en cas d'ostéochondrose
traitement local de douleurs en cas de raideur articulaire, de préférence en cas de fibrose articulaire ou d'épaule gelée ;
traitement local de douleurs musculaires, par injection intramusculaire, par exemple en cas d'une déchirure musculaire, de courbatures ou d'affections spastiques;
injection locale dans le ménisque douloureux en cas de dégénérescence du ménisque ou de rupture du ménisque,
traitement de douleurs dorsales par injection dans le disque intervertébral en cas de dégénérescence du disque intervertébral ou de rupture du disque intervertébral ;
injection autour d'un nerf douloureux, par ex. en cas de névralgie du trijumeau, neurinome, névrome de Morton, douleur fantôme ou névrome cicatriciel ;
traitement intra/péridentale de douleurs dentaires par utilisation locale en cas de :
carie
toutes formes de douleur dentaire
avant/pendant/après une extraction dentaire
avant/pendant/après une pose d'implant dentaire
utilisation locale en cas de parodontite
utilisation locale en cas de collets dentaires dénudés ;
injection dans la cavité pleurale en cas de douleurs pleurétiques ; instillation dans l'intestin en cas de douleurs intestinales, en particulier en cas de colite ulcéreuse, maladie de Crohn ou fissure anale.

25. Agent pour l'utilisation selon une des revendications 21 à 24, **caractérisé en ce que** l'agent est injecté dans une cavité synoviale qui n'est pas revêtue d'urothélium.

26. Résinifératoxine pour l'utilisation dans un agent pour le traitement de
arthrose
arthrite, en particulier polyarthrite rhumatoïde et arthrite infectieuse chondrocalcinose
lésions ligamentaires
lésion du ménisque
lésion cartilagineuse
synovite
fibrose articulaire
maladie de Sudeck
nécrose des parties articulaires
douleurs articulaires neuropathiques ;
traitement de douleurs osseuses après chirurgie osseuse par application sur l'os, par ex. après
une ostéotomie de la crête iliaque
une correction d'un hallux valgus;
traitement de douleurs osseuses par injection dans l'os
par ex. dans la tête fémorale en cas de nécrose de la tête fémorale dans le corps vertébral en cas d'ostéochondrose
traitement de douleurs en cas de raideur articulaire, en particulier
fibrose articulaire
épaule gelée
traitement de douleurs musculaires, par injection intramusculaire, par exemple en cas de
déchirure musculaire
courbatures
affections spastiques;
injection dans le ménisque douloureux en cas de dégénérescence du ménisque ou de rupture du ménisque
traitement de douleurs dorsales par injection dans le disque intervertébral en cas de dégénérescence du disque intervertébral ou de rupture du disque intervertébral ;
injection autour d'un nerf douloureux, par ex. en cas de
névralgie du trijumeau
neurinome
névrome de Morton
douleur fantôme
névrome cicatriciel ;
traitement intra/péridentale de douleurs dentaires en cas de :
carie
toutes formes de douleur dentaire
avant/pendant/après une extraction dentaire
avant/pendant/après une pose d'implant dentaire
utilisation en cas de parodontite
utilisation en cas de collets dentaires dénudés ;
injection dans la cavité pleurale en cas de douleurs pleurétiques ;
instillation dans l'intestin en cas de douleurs intestinales, en particulier colite ulcéreuse, maladie de Crohn, fissure anale, hémorroïdes ;
douleurs articulaires ;
douleurs osseuses après une ostéotomie ;
épaule gelée ;
tendinite
myalgie ;
douleurs en cas de tumeurs de tissus mous ;
douleurs osseuses ou
douleurs ostéo-articulaires ;
**caractérisée en ce que**
A) l'agent contient en plus un sel de calcium ;
B) la concentration en ions calcium est supérieure à 2 mmol ;
C) l'agent est dissous dans une solution tampon ayant un pH supérieur à 7,6, de préférence supérieur à 8,5 ; et
D) l'agent ne contient pas d'anesthésique local.
